# FASCICULE DE BREVET EUROPEEN

(11) **EP 1 051 243 B1**
(45) Date de publication et mention de la délivrance du brevet: **12.05.2004**
(21) Numéro de dépôt: 99900987.1
(22) Date de dépôt: 22.01.1999
(51) Int. Cl.: B01J 13/02

(54) **GRANULES DISPERSABLES DANS L'EAU COMPRENANT UNE MATIERE ACTIVE HYDROPHOBE**
WASSERABWEISENDEN WIRKSTOFF ENTHALTENDE UND WASSERDISPERGIERBARE KÖRNCHEN
WATER DISPERSIBLE GRANULATES COMPRISING AN ACTIVE HYDROPHOBIC SUBSTANCE

(30) Priorité: 02.02.1998 FR 9801158
(43) Date de publication de la demande: 15.11.2000
(73) Titulaire: RHODIA CHIMIE, 92408 Courbevoie Cédex (FR)
(72) Inventeur: GUERIN, Gilles, F-95600 Eaubonne (FR); MORVAN, Mikel, F-92400 Courbevoie (FR); TAQUET, Pascal, F-60340 Villers-sous-Saint-Leu (FR)
(74) Mandataire: Fabre, Madeleine-France
(86) Numéro de dépôt international: PCT/FR1999/000131
(87) Numéro de publication internationale: WO 1999/038611

(56) Documents cités:
- EP-A- 0 371 878
- EP-A- 0 511 037
- EP-A- 0 609 121
- EP-A- 0 633 310

## Description

La présente invention a pour objet des granulés dispersables dans l'eau comprenant une matière active hydrophobe, notamment sous forme liquide, leur procédé de préparation et leur utilisation.

Dans certains domaines, comme par exemple ceux de l'alimentaire, de la cosmétique, de l'agrochimie ou des peintures, on est conduit à préparer des formulations à partir de matières actives sous la forme de liquide hydrophobe. L'une des possibilités de formulation est de préparer des émulsions huile dans eau de telles matières.

Cependant, on se trouve en face de problèmes liés à la stabilité au stockage de ces émulsions. En effet, il est courant de constater une séparation de phases plus ou moins importante des constituants de l'émulsion. En outre, on peut être confronté à des problèmes de désactivation de la matière active, ladite matière active pouvant se dégrader par hydrolyse lors du stockage.

Enfin, il n'est pas à négliger le fait que pour être facilement manipulables et pompables, de telles formulations présentent des teneurs relativement peu élevées en matière active et une quantité d'eau importante.

En ce qui concerne certaines matières actives hydrophobes solides, peuvent se poser des problèmes de difficultés de manipulation dues à l'envolement desdites matières actives, ou de sensibilité vis-à-vis du milieu environnant.

La présente invention a donc pour objectif de proposer une alternative originale aux problèmes mentionnés ci-dessus en ce sens que les formulations proposées sont des poudres contenant des concentrations élevées en matière active, notamment sous forme de liquide hydrophobe.

La Demanderesse a déjà présenté une solution originale à ces problèmes en proposant des granulés dispersables dans l'eau, obtenus par séchage d'une émulsion huile dans eau comprenant une matière active hydrophobe liquide, un composé hydrosoluble un agent tensioactif et de l'eau, le composé hydrosoluble étant susceptible de former par séchage un film solide emprisonnant les goutelettes de matière active (demande internationale WO 97/15387, dans laquelle le composé hydrosoluble est en un sucre ; demande internationale WO 97/15386, dans laquelle le composé hydrosoluble est en urée, un sucre, un polyélectrolyte, un polycondensat synthétique d'acide aminé ; demande internationale WO 97/15385, dans laquelle l'agent tensioactif est non-ionique choisi parmi les dérivés polyoxyalkylénés, et le composé hydrosoluble choisi parmi les polyélectrolytes appartenant à la famille des polyacides faibles, comme les polymères peptidiques dérivés de la polycondensation d'acides aminés. La demande WO 98/39401 décrit des compositions détergentes en poudre comprenant un silicone aminé, des agents tensioactifs et divers additifs de détergence, notamment des agents « builders » comme par exemple de l'acide polyaspartique, de l'acide polyglutamique ou leurs leurs sels, des polyimides dérivés de la polycondensations de lacide aspartique et/ou glutamique.

Ainsi, elle a pour objet des granulés dispersables dans l'eau comprenant :
- au moins une matière active organique hydrophobe (MA) solide ou de point de fusion inférieur à 100°C, sous forme de particules, finement divisée dans et encapsulée par une matrice solide en une protéine (PP) hydrosoluble ou hydrodispersable d'origine d'origine végétale ;
- et au moins un dispersant ionique ou amphotère (D) à l'interface matière active/matrice.
Dans sa demande publiée sous le n° WO 99/38945, déposée le même jour, la Demanderesse décrit des granulés similaires, comprenant un parfum hydrophobe.
Dans sa demande publiée sous le n° WO 99/38911, déposée le même jour, la Demanderesse décrit des granulés similaires, comprenant un silicone aminé liquide, comme matière active hydrophobe.

Pour une bonne réalisation de l'invention, lesdits granulés dispersables dans l'eau, comprennent
- de 5 à 90 %, de préférence de 40 à 85 %, tout particulièrement de 50 à 80 % de leur poids en matière active hydrophobe organique (MA),
- de 3 à 90 %, de préférence de 10 à à 60%, tout particulièrement de 15 à 50% de leur poids en une protéine d'origine végétale (PP) hydrosoluble ou hydrodispersable
- de 0,02 à 20 %, de préférence de 0,1 à 10 % de leur poids, en agent dispersant (D) ionique ou amphotère,
lesdits pourcentages étant exprimés en poids de matière sèche.

Lesdites particules de matière active (MA) peuvent présenter une granulométrie moyenne de l'ordre de 0,1 à 50µm, de préférence de l'ordre de 0,1 à 10 µm, tout particulièrement de 0,2 à 5µm.

Toutes les matières actives, qu'elles soient solides, liquides (telles quelles ou en solution dans un solvant) conviennent à l'invention, dans la mesure où elles ne sont pas miscibles ou ne sont que très faiblement miscibles à l'eau.

Par faiblement miscible, on entend des matières actives dont la solubilité dans l'eau à pH 7 ne dépasse pas 10 % en poids.

D'une manière préférentielle, ladite matière active organique hydrophobe a un point de fusion inférieur à 100°C ; tout particulièrement, celle-ci est liquide à température ordinaire.

Par la suite, on entendra par matière active, soit la matière active pure (qui peut elle-même être un solvant) ou un mélange de matières actives, soit la matière active (ou un mélange de matières actives) solubilisée dans un solvant.

A titre d'exemple de matières actives utilisées dans le domaine de l'alimentaire, on peut citer les mono-, di- et triglycérides, les huiles essentielles, les arômes, les colorants.

A titre d'exemple de matières actives utilisées dans le domaine de la cosmétique on peut citer les huiles silicones appartenant par exemple à la famille des diméthicones.

A titre d'exemple de matières actives utilisées dans le domaine des peintures, on peut citer les résines alkydes, les résines époxy, les isocyanates bloqués ou non.

Dans le domaine du papier, on peut citer à titre d'exemple les résines de collage et d'hydrofugation telles que le dimère d'alkylcétène (AKD) ou l'anhydride alcényle succinique (ASA).

Dans le domaine de la détergence, on peut mentionner en tant que matière active possible les antimousses silicones.

Dans le domaine du bâtiment, on peut citer des produits d'hydrofugation, comme les organopolysiloxanes linéaires, cycliques et/ou ramifiés pouvant comporter dans la chaîne et/ou en extrêmité de chaîne des fonctions réactives hydroxy, alcoxy ...

Dans le domaine des matières actives phytosanitaires, on peut mentionner des fongicides (iprodione ...), des herbicides (isoproturon), des insecticides (pyrethroides de synthèse ...)

Il est de même possible d'utiliser des matières actives telles que les lubrifiants pour le travail ou la déformation des matériaux.

Lorsque la matière active est un solvant (ou un mélange de solvants) ou une solution dans un solvant (ou plusieurs solvants), ledit solvant n'est pas ou n'est que peu miscible dans l'eau au sens indiqué précédemment. A titre d'exemples, on peut citer les solvants mis en oeuvre pour le nettoyage ou le décapage, tels que les coupes pétrolières aromatiques, les composés terpéniques comme le D-limonène, ou encore le L-limonène, ainsi que les solvants comme le Solvesso®, les esters aliphatiques, comme les esters méthyliques d'un mélange d'acides acétique, succinique et glutarique (mélange d'acides sous-produit de la synthèse du Nylon), les huiles paraffiniques comme l'huile de vaseline, les alcanes, les solvants chlorés, les triglycérides de synthèse ou naturels.

Les protéines d'origine végétale sont de préférence hydrolysées, avec un degré d'hydrolyse inférieur ou égal à 40%, par exemple de 5 à moins de 40%.

Parmi les protéines d'origine végétale, on peut mentionner à titre indicatif les protéines provenant des graines protéagineuses notamment celles de pois, de féverole, de lupin, de haricot, et de lentille ; les protéines provenant de grains de céréales notamment celles du blé, de l'orge, du seigle, du maïs, du riz, de l'avoine, et du millet ; les protéines provenant des graines oléagineuses notamment celles du soja, de l'arachide, du tournesol, du colza, et de la noix de coco ; les protéines provenant des feuilles notamment de luzerne, et d'orties ; et les protéines provenant d'organes végétaux de réserves enterrées notamment celle de pomme de terre, et de betterave.

D'une manière préférentielle, la protéine d'origine végétale est un hydrolysat de protéine provenant du soja ou du blé.

Les granulés redispersables de la présente invention contiennent un dispersant ionique ou amphotère (D).

Parmi les dispersants anioniques, on peut citer
. des émulsifiants comme les sels hydrosolubles d'alkylsulfates, d'alkyléthersulfates, les alkyliséthionates et les alkyltaurates ou leurs sels, les alkylcarboxylates, les alkylsulfosuccinates ou les alkylsuccinamates, les alkylsarcosinates, les dérivés alkylés d'hydrolysats de protéines, les acylaspartates, les phosphates esters d'alkyle et/ou d'alkyléther et/ou d'alkylaryléther.
   Le cation est en général un métal alcalin ou alcalino-terreux, tels que le sodium, le potassium, le lithium, le magnésium, ou un groupement ammonium NR₄⁺ avec R, identiques ou différents, représentant un radical alkyle substitué ou non par un atome d'oxygène ou d'azote.
. des dispersants comme les lignosulfonates, les polynaphtalène sulfonates , les copolymères comprenant des motifs dérivés d'au moins un monomère choisi parmi les acides, les diacides ou les anhydrides insaturés en C₃-C₅ et des motifs dérivés d'au moins un hydrocarbure insaturé linéaire ou ramifié en C₄-C₈.

Parmi les dispersants cationiques, on peut citer des émulsifiants comme les halogénures d'alkyldiméthylbenzylammonium, les halogénures d'alkyldiméthyléthylammonium ...

Parmi les dispersants amphotères, on peut citer des émulsifiants comme les alkyl-bétaïnes, les alkyldiméthylbétaïnes, les alkylamidopropylbétaines, les alkylamidopropyldiméthylbétaïnes, les alkyltriméthyl-sulfobétaïnes, les dérivés d'imidazoline tels que les alkylamphoacétates, alkylamphodiacétates, alkylampho-propionates, alkylamphodipropionates, les alkylsultaïnes ou les alkylamidopropyl-hydroxysultaïnes, les produits de condensation d'acides gras et d'hydrolysats de protéines, les dérivés amphotères des alkylpolyamines comme l'Amphionic XL® commercialisé par Rhône-Poulenc, Ampholac 7T/X® et Ampholac 7C/X® commercialisés par Berol Nobel, les protéines ou hydrolystas de protéines.

D'une manière préférentielle, ledit dispersant (D) est une protéine ou un hydrolysat de protéine ; il peut être constitué par la protéine d'origine végétale de la matrice.

Selon une variante de réalisation de l'invention, jusqu'à 95%, de préférence jusqu'à 50 % du poids de la protéine d'origine végétale constituant la matrice, est remplacé par un ose, un oside ou un polyholoside hydrosoluble ou hydrodispersable (O) ou un polyelectrolyte (PE) appartenant à la famille des polyacides faibles.

Parmi les oses on peut mentionner les aldoses tels que le glucose, le mannose, le galactose, le ribose, et les cétoses tels que le fructose.

Les osides sont des composés qui résultent de la condensation, avec élimination d'eau, de molécules d'oses entre elles ou encore de molécules d'oses avec des molécules non glucidiques. parmi les osides on préfère les holosides qui sont formés par la réunion de motifs exclusivement glucidiques et plus particulièrement les oligoholosides (ou oligosaccharides) qui ne comportent qu'un nombre restreint de ces motifs, c'est-à-dire un nombre en général inférieur ou égal à 10. A titre d'exemples d'oligoholosides, on peut mentionner le saccharose, le lactose, la cellobiose, le maltose et le tréhalose.

Les polyholosides (ou polysaccharides) fortement dépolymérisés convenables sont décrits par exemple dans l'ouvrage de P. ARNAUD intitulé "cours de chimie organique", Gaultier-Villars éditeurs, 1987. Plus particulièrement, ces polyholosides ont une masse moléculaire en poids inférieure à 20 000 g/mole.

A titre d'exemple non limitatif de polyholosides fortement dépolymérisés, on peut citer le dextran, l'amidon, la gomme xanthane et les galactomannanes tels que le guar ou la caroube, ces polysaccharides présentant de préférence un point de fusion supérieur à 100°C et une solubilité dans l'eau comprise entre 50 et 500g/l.

Le polyélectrolyte (PE) peut être choisi parmi ceux issus de la polymérisation de monomères de formule générale

(R¹)(R²)C=C(R³) COOH

formule dans laquelle R¹, R², et R³ sont identiques ou différents et représentent un atome d'hydrogène,
. un radical hydrocarboné contenant de 1 à 4 atomes de carbone, méthyle de préférence
. une fonction -COOH
. un radical -R-COOH, où R représente un reste hydrocarboné contenant de 1 à 4 atomes de carbone, de préférence un reste alkylène contenant 1 ou 2 atomes de carbone, méthylène tout particulièrement.

A titre d'exemples non limitatifs, on peut citer les acides acrylique, méthacrylique, maléique, fumarique, itaconique, crotonique.

Conviennent également les copolymères obtenus à partir des monomères répondant à la formule générale précédente et ceux obtenus à l'aide de ces monomères et d'autres monomères, en particulier les dérivés vinyliques comme les alcools vinyliques et les amides copolymérisables comme l'acrylamide ou le méthacrylamide. On peut également citer les copolymères obtenus à partir d'alkyle vinyl éther et d'acide maléique ainsi que ceux obtenus à partir de vinyl styrène et d'acide maléique qui sont notamment décrits dans l'encyclopédie KIRK-OTHMER intitulé "ENCYCLOPEDIA OF CHEMICAL TECHNOLOGY" - Volume 18 - 3 ème édition - Wiley interscience publication - 1982.

Les polyélectrolytes préférés présentent un degré de polymérisation faible. La masse moléculaire en poids des polyélectrolytes est plus particulièrement inférieure à 20000 g/mole. De préférence, elle est comprise entre 1000 et 5000 g/mole.

Les granulés selon la présente invention présentent de nombreux avantages. Tout d'abord, leur mise en forme permet d'éviter tous les problèmes dus à l'emploi d'émulsions lorsque la matière active (MA) est liquide. Ainsi, les problèmes d'instabilité au stockage sont évités, tels que notamment le crémage, la floculation, le mûrissement et la coalescence. Ces différents phénomènes sont décrits dans "ENCYCLOPEDIA of EMULSIONS TECHNOLOGY", volume 1 par Paul BECHER aux éditions MARCEL DEKKER INC., 1983.

Un autre avantage de la présente invention est que les concentrations en matières actives liquides peuvent être très élevées. Par conséquent, lors de l'utilisation de ces granulés, une faible quantité de granulés sera suffisante.

Par ailleurs, la mise en forme selon l'invention permet de protéger ladite matière active liquide ou solide, lorsque celle-ci est fragile (sensibilité à l'hydrolyse, volatilité ...).

La présente invention permet aussi de disposer d'une préformulation sous forme de granulés, donc plus facilement utilisable que des émulsions par exemple dans le cas où ladite préformulation est ajoutée à une formulation en poudre.

En outre, les granulés selon l'invention, en raison de la présence du dispersant (D) présentent l'avantage de se disperser dans l'eau, pour donner une émulsion.

Enfin, la présente invention propose une solution aux problèmes de la mise en forme de produits liquides qui sont habituellement formulés par absorption sur un support. Ces formulations sont souvent peu concentrées en matière active et il peut se produire une séparation de phases entre le support et la matière active par migration de la matière active au cours du stockage.

Un deuxième objet de l'invention consiste en un procédé de préparation en deux étapes, desdits granulés dispersables dans l'eau,
- la première étape consistant à préparer une dispersion dans l'eau comprenant au moins une matière active hydrophobe, au moins un dispersant ionique ou amphotère (D) et au moins une protéine (PP) hydrosoluble ou hydrodispersable d'origine végétale,
- la deuxième étape consistant à sécher ladite dispersion jusqu'à formation d'un granulé.

Les quantités relatives de matière active hydrophobe (MA), de dispersant ionique ou amphotère (D) et de protéine d'origine végétale (PP) mises en oeuvre étant telles que ladite dispersion, exprimée en sec, comprenne :
- de 5 à 90 %, de préférence de 40 à 85 %, tout particulièrement de 50 à 80 % de son poids en matière active hydrophobe organique (MA),
- de 3 à 90 %, de préférence de 10 à 60%, tout particulièrement de 15 à 50% de son poids en protéine d'origine végétale (PP) hydrosoluble ou hydrodispersable
- de 0,02 à 20 %, de préférence de 0,1 à 10 % de son poids, en agent dispersant (D) ionique ou amphotère,
lesdits pourcentages étant exprimés en poids de matière sèche.

La quantité de matière sèche de la dispersion est généralement comprise entre 10 et 70 % en poids et préférentiellement entre 30 et 60% en poids.

Lorsque la matière active (MA) est solide, on peut réaliser une dispersion (suspension) concentrée en introduisant sous agitation la matière active (MA) dans une solution aqueuse contenant l'agent dispersant (D) et la protéine d'origine végétale hydrosoluble ou hydrodispersable. La suspension concentrée ainsi préparée, dont la taille moyenne de particules peut aller de 1µm à 50µm, peut être broyée pour obtenir une bouillie présentant une taille moyenne de particules comprise entre 1µm et 5µm et une viscosité (Brookfield 20r.p.m. à 25°C) inférieure à 1000 mPa.s.

Lorsque la matière active (MA) présente un point de fusion inférieur à 100°C ou bien se présente sous forme liquide à température ambiante, on peut mettre en oeuvre, à la température adaptée, toutes les méthodes de préparation d'émulsions connues de l'homme du métier et qui sont décrites dans "ENCYCLOPEDIA of EMULSIONS TECHNOLOGY", volumes 1 à 3 de Paul BECHER édités par MARCEL DEKKER INC., 1983.

Ainsi, la méthode dite d'émulsification en phase directe convient à la préparation des granulés selon l'invention. Il est rappelé brièvement que cette méthode consiste à préparer un mélange contenant l'eau, l'agent dispersant (D), la protéine d'origine végétale hydrosoluble ou hydrodispersable, puis à introduire la matière active sous forme liquide, sous agitation.

On peut aussi préparer l'émulsion en mettant en oeuvre des broyeurs colloïdaux tels que MENTON GAULIN et MICROFLUIDIZER (MICROFLUIDICS).

La granulométrie moyenne de l'émulsion est en général comprise entre 0,1 et 10 micromètres et préférentiellement entre 0,2 et 5 micromètres.

L'émulsification peut être réalisée à une température voisine de la température ambiante, bien que des températures plus faibles ou plus élevées soient envisageables.

La seconde étape du procédé de préparation selon l'invention consiste à sécher la dispersion (suspension ou émulsion) ainsi formulée pour obtenir des granulés.

La méthode mise en oeuvre pour éliminer l'eau de la dispersion et obtenir des granulés peut être effectuée par tout moyen connu de l'homme du métier.

Conviennent par exemple la lyophilisation, qui correspond à une étape de congélation, suivie d'une étape de sublimation, ou bien encore le séchage par atomisation.

Ces modes de séchage, et plus particulièrement celui par atomisation, sont particulièrement indiqués car ils permettent de conserver la dispersion en l'état et d'obtenir directement des granulés. Les protéines d'origine végétale hydrosolubles ou hydrodispersables sont particulièrement bien adaptées au séchage par atomisation, car elles sont particulièrement stables en température.

Le séchage par atomisation peut s'effectuer de manière habituelle dans tout appareil connu tel que par exemple une tour d'atomisation associant une pulvérisation réalisée par une buse ou une turbine avec un courant de gaz chaud.
Les conditions de mise en oeuvre sont fonction de la nature de la matrice, de la thermosensibilité de la matière active et de l'atomiseur utilisé ; ces conditions sont généralement telles que la température de l'ensemble du produit au cours du séchage ne dépasse pas 150°C, de préférence ne dépasse pas 110°C.

Les granulés obtenus sont redispersables dans l'eau, pour donner à nouveau une dispersion présentant généralement une granulométrie voisine de celle de la dispersion initiale.

Il est à noter que des additifs, tels que les agents antimottants peuvent être incorporés aux granulés au moment de cette seconde étape de séchage. On recommande d'utiliser une charge choisie notamment parmi le carbonate de calcium, le sulfate de baryum, le kaolin, la silice, la bentonite, l'oxyde de titane, le talc, l'alumine hydratée et le sulfoaluminate de calcium.

Les exemples suivants sont donnés à titre illustratif.

### Exemple 1 :

### - Réalisation d'une émulsion d'huile silicone dans une solution aqueuse de protéine de soja -

On prépare un mélange de composition suivante

| | |
|---|---|
| FP940 (hydrolysat de proteine de soja de degré d'hydrolyse inférieur à 5%, de Protein Technologies International) | 1,8 partie en poids (en sec) |
| Huile silicone | 30 parties en poids |
| Eau permutée | 68,2 parties en poids |

par addition d'huile silicone à une solution aqueuse à 5% en poids de FP940.
Le taux de matière séche est de 31,8%.
Le mélange est d'abord pré-émulsionné à l'aide d'un de l'appareil Ultra Turrax T25 durant 1 minute à 9500 t/mn.
L'émulsion proprement dite est réalisée au moyen d'un microfluidiseur (M110T de Microfluidics) dans les conditions suivantes : Pression : 600 bars - 3 passages dans le microfluidiseur - Bain d'eau froide en sortie du microfluidiseur.
L'émulsion obtenue présente une granulométrie resserrée avec un diamètre médian (d50) de 1 µm.

### - Incorporation de la matrice -

On incorpore à l'émulsion préparée une protéine végétale hydrolysée à 15% (FP900 de Protein Technologies International) comme matrice polypeptidique. La composition de l'émulsion formulée est la suivante :

| | |
|---|---|
| FP940 (hydrolysat de proteine de soja de degré d'hydrolyse inférieur à 5%, de Protein Technologies International) (émulsifiant) | 1,5 partie en poids (en sec) |
| Huile silicone | 24,75 parties en poids |
| FP900 (hydrolysat de proteine de soja de degré d'hydrolyse de 15%, de Protein Technologies International) (matrice) | 17,5 parties en poids (en sec) |
| Eau permutée | 56,25 parties en poids |

Cette émulsion formulée présente 43,75 % de matière sèche et un diamètre médian (d50) de 1 µm.
La composition de cette émulsion correspond à un rapport pondéral en sec A/B de 60/40, rapport dans lequel A et B ont la signification suivante :
A = (huile silicone+proteine FP940) / % total matière sèche X 100
B = proteine FP900 / % total matière sèche X100

### - Séchage de l'émulsion formulée :

Cette émulsion est ensuite séchée par lyophilisation. Les granulés issus de ce traitement ont la composition suivante :

| | |
|---|---|
| FP940 (hydrolysat de proteine de soja de degré d'hydrolyse inférieur à 5%, de Protein Technologies International) (émulsifiant) | 3,4 parties en poids |
| Huile silicone | 56,6 parties en poids |
| FP900 (hydrolysat de proteine de soja de degré d'hydrolyse de 15%, de Protein Technologies International) (matrice) | 40 parties en poids |

La redispersion des granulés dans l'eau donne à nouveau une émulsion d'huile silicone de distribution granulométrique assez homogène et de diamètre médian (d50) de 8 µm.

### Exemple 2 :

On répète les opérations décrites à l'exemple 1, en méttant en oeuvre
- à l'étape de réalisation de l'émulsion, une huile de vaseline (au lieu d'une huile silicone), selon les proportions suivantes :

| | |
|---|---|
| FP940 (hydrolysat de proteine de soja de degré d'hydrolyse inférieur à 5%, de Protein Technologies International ) | 0,6 partie en poids |
| Huile de vaseline | 29,6 parties en poids |
| Eau permutée | 69,8 parties en poids |

L'émulsion obtenue présente un taux de matière séche est de 30,2% et une granulométrie de diamètre médian (d50) de 1,3 µm.
- à l'étape d'incorporation de la matrice, un mélange de protéine FP900 et de saccharose selon un rapport pondéral 15/85 (au lieu de protéine FP900 seule), selon les proportions suivantes :

| | |
|---|---|
| FP940 (hydrolysat de proteine de soja de degré d'hydrolyse inférieur à 5%, de Protein Technologies International (émulsifiant) | 0,55 partie en poids (en sec) |
| Huile de vaseline | 27,5 parties en poids |
| FP900 (hydrolysat de proteine de soja de degré d'hydrolyse de 15%, de Protein Technologies International) (matrice) | 1,05 partie en poids (en sec) |
| Saccharose (matrice) | 6 parties en poids |
| Eau permutée | 64,9 parties en poids |

Cette émulsion formulée présente 35,1 % de matière sèche et un diamètre médian (d50) de 1 µm.
Le rapport A/B est de 80/20, avec
A= huile de vaseline + proteine FP940 / % total matière sèche X 100
B= proteine FP900 + saccharose / % total matière sèche X 100
Les granulés obtenus après lyophilisation ont la composition suivante :

| | |
|---|---|
| FP940 (hydrolysat de proteine de soja de degré d'hydrolyse inférieur à 5%, de Protein Technologies International ) (émulsifiant) | 1,55 partie en poids |
| Huile de vaseline | 78,35 parties en poids |
| FP900 (hydrolysat de proteine de soja de degré d'hydrolyse de 15%, de Protein Technologies International ) (matrice) | 3 parties en poids |
| Saccharose (matrice) | 17,1 parties en poids |

La redispersion des granulés dans l'eau donne à nouveau une émulsion d'huile de vaseline de distribution granulométrique ressérée avec un diamètre médian (d50) de 2,5 µm.

## Revendications

1. Granulés dispersables dans l'eau comprenant :
- au moins une matière active organique hydrophobe (MA) solide ou de point de fusion inférieur à 100°C, sous forme de particules, finement divisée dans et encapsulée par une matrice solide en une protéine (PP) hydrosoluble ou hydrodispersable d'origine d'origine végétale ;
- et au moins un dispersant ionique ou amphotère (D) à l'interface matière active/matrice.

2. Granulés selon la revendication 1), **caractérisés en ce qu'**ils comprennent
- de 5 à 90 %, de préférence de 40 à 85 %, tout particulièrement de 50 à 80 % de leur poids en matière active hydrophobe organique (MA),
- de 3 à 90 %, de préférence de 10 à 60%, tout particulièrement de 15 à 50% de leur poids en protéine (PP) hydrosoluble ou hydrodispersable
- de 0,02 à 20 %, de préférence de 0,1 à 10 % de leur poids, en agent dispersant (D) ionique ou amphotère,
lesdits pourcentages étant exprimés en poids de matière sèche.

3. Granulés selon la revendication 1) ou 2), **caractérisés en ce que** les particules de matière active (MA) présentent une granulométrie moyenne de l'ordre de 0,1 à 50µm, de préférence de l'ordre de 0,1 à 10 µm, tout particulièrement de 0,2 à 5µm.

4. Granulés selon l'une quelconque des revendications 1) à 3), **caractérisés en ce que** la matière active organique hydrophobe est liquide à température ordinaire.

5. Granulés selon l'une quelconque des revendications 1) à 4), **caractérisés en ce que** la matière active organique hydrophobe est une matière active du domaine de l'alimentaire, du domaine de la cosmétique, du domaine des peintures, du domaine du papier, du domaine de la détergence, du domaine du bâtiment, du domaine des matières actives phytosanitaires, ou un lubrifiant pour le travail ou la déformation des matériaux.

6. Granulés selon l'une quelconque des revendications 1) à 5), **caractérisés en ce que** la protéine (PP) est une protéine d'origine végétale hydrolysée avec un degré d'hydrolyse inférieur à 40%.

7. Granulés selon la revendication 6), **caractérisés en ce que** la protéine est un hydrolysat de protéine provenant du soja ou du blé.

8. Granulés selon l'une quelconque des revendications 1) à 7), **caractérisés en ce que** le dispersant (D) est anionique et choisi parmi les sels hydrosolubles d'alkylsulfates, d'alkyléthersulfates, les alkyliséthionates et les alkyltaurates ou leurs sels, les alkylcarboxylates, les alkylsulfosuccinates ou les alkylsuccinamates, les alkylsarcosinates, les dérivés alkylés d'hydrolysats de protéines, les acylaspartates, les phosphates esters d'alkyle et/ou d'alkyléther et/ou d'alkylaryléthe, les lignosulfonates, les polynaphtalène sulfonates , les copolymères comprenant des motifs dérivés d'au moins un monomère choisi parmi les acides, les diacides ou les anhydrides insaturés en C₃-C₅ et des motifs dérivés d'au moins un hydrocarbure insaturé linéaire ou ramifié en C₄-C₈.

9. Granulés selon l'une quelconque des revendications 1) à 7), **caractérisés en ce que** le dispersant (D) est cationique et choisi parmi les halogénures d'alkyldiméthylbenzylammonium, les halogénures d'alkyldiméthyléthyl-ammonium.

10. Granulés selon l'une quelconque des revendications 1) à 7), **caractérisés en ce que** le dispersant (D) est amphotère et choisis parmi les alkyl-bétaïnes, les alkyldiméthylbétaïnes, les alkylamidopropylbétaines, les alkylamidopropyldiméthylbétaïnes, les alkyltriméthyl-sulfobétaïnes, les dérivés d'imidazoline tels que les alkylamphoacétates, alkylamphodiacétates, alkylampho-propionates, alkylamphodipropionates, les alkylsultaïnes ou les alkylamidopropyl-hydroxysultaïnes, les produits de condensation d'acides gras et d'hydrolysats de protéines, les dérivés amphotères des alkylpolyamines, les protéines ou hydrolystas de protéines.

11. Granulés selon la revendication 10), **caractérisés en ce que** le dispersant (D) est une protéine ou un hydrolysat de protéine.

12. Granulés selon l'une quelconque des revendications 1) à 11), **caractérisés en ce que** jusqu'à 95%, de préférence jusqu'à 50 % du poids de la protéine d'origine végétale (PP) constituant la matrice, est remplacé par un ose, un oside ou un polyholoside hydrosoluble ou hydrodispersable (O) ou un polyelectrolyte (PE) appartenant à la famille des polyacides faibles.

13. Procédé de préparation en deux étapes, de granulés dispersables dans l'eau faisant l'objet de la revendication 1),
- la première étape consistant à préparer une dispersion dans l'eau comprenant au moins une matière active hydrophobe, au moins un dispersant ionique ou amphotère (D) et au moins une protéine (PP) hydrosoluble ou hydrodispersable d'origine végétale,
- la deuxième étape consistant à sécher ladite dispersion jusqu'à formation d'un granulé.

14. Procédé selon la revendication 13), **caractérisé en ce que** les quantités relatives de matière active hydrophobe (MA), de dispersant ionique ou amphotère (D) et de protéine d'origine végétale (PP) hydrosoluble ou hydrodispersable mises en oeuvre sont telles que ladite dispersion, exprimée en sec, comprenne
- de 5 à 90 %, de préférence de 40 à 85 %, tout particulièrement de 50 à 80 % de son poids en matière active hydrophobe organique (MA),
- de 3 à 90 %, de préférence de 10 à 60 %, tout particulièrement de 15 à 50% de son poids en protéine d'origine végétale (PP) hydrosoluble ou hydrodispersable
- de 0,02 à 20 %, de préférence de 0,1 à 10 % de son poids, en agent dispersant (D) ionique ou amphotère, lesdits pourcentages étant exprimés en poids de matière sèche.

15. Procédé selon la revendication 13) ou 14), **caractérisé en ce que** la quantité de matière sèche de la dispersion est comprise entre 10 et 70 % en poids et préférentiellement entre 30 et 60% en poids.

16. Procédé selon l'une quelconque des revendications 13) à 15), **caractérisé en ce que** la dispersion est une suspension de matière active (MA) solide, dont la taille moyenne de particules est comprise entre 1µm et 50µm, de préférence entre 1µm et 5µm et de viscosité (Brookfield 20r.p.m. à 25°C) inférieure à 1000 mPa.s.

17. Procédé selon l'une quelconque des revendications 13) à 15), **caractérisé en ce que** la dispersion est une émulsion de matière active (MA) liquide, dont la taille moyenne de particules est comprise entre 0,11µm et 10µm, de préférence entre 0,2µm et 5µm.

18. Procédé selon l'une quelconque des revendications 13 à 17), **caractérisé en ce que** la matière active organique hydrophobe (MA), la protéine d'origine végétale (PP) hydrosoluble ou hydrodispersable, le dispersant ionique ou amphotère (D) sont choisis parmi ceux mentionnés aux revendications 5), 6), 7), 8), 9), 10), 11), ou 12).

19. Procédé selon l'une quelconque des revendications 13) à 18, **caractérisé en ce que** le séchage de la dispersion est réalisé par lyophilisation ou par atomisation.

20. Procédé selon l'une quelconque des revendications 13) à 18, **caractérisé en ce que** des agents antimottants sont introduits pendant l'étape de séchage.

## Claims

1. Water-dispersible granules comprising:
- at least one hydrophobic organic active material (AM) which is solid or has a melting point of less than 100°C, in the form of particles, which is finely divided in and encapsulated by a solid matrix made of a water-soluble or water-dispersible protein (PP) of plant origin;
- and at least one ionic or amphoteric dispersant (D) at the active material/matrix interface.

2. Granules according to Claim 1, **characterized in that** they comprise
- from 5% to 90%, preferably from 40% to 85% and most particularly from 50% to 80%, of their weight of organic hydrophobic active material (AM),
- from 3% to 90%, preferably from 10% to 60% and most particularly from 15% to 50%, of their weight of water-soluble or water-dispersible protein (PP)
- from 0.02% to 20%, preferably from 0.1% to 10%, of their weight of ionic or amphoteric dispersant (D), the said percentages being expressed by weight of solids.

3. Granules according to Claim 1 or 2, **characterized in that** the particles of active material (AM) have a mean particle size from about 0.1 µm to 50 µm, preferably from about 0.1 µm to 10 µm and most particularly from 0.2 µm to 5 µm.

4. Granules according to any one of Claims 1 to 3, **characterized in that** the hydrophobic organic active material is liquid at ordinary temperature.

5. Granules according to any one of Claims 1 to 4, **characterized in that** the hydrophobic organic active material is an active material from the food sector, from the cosmetics sector, from the paints sector, from the paper sector, from the detergency sector, from the construction sector, from the plant-protection active materials sector or a lubricant for working or deforming materials.

6. Granules according to any one of Claims 1 to 5, **characterized in that** the protein (PP) is a protein of plant origin, hydrolysed with a degree of hydrolysis of less than 40%.

7. Granules according to Claim 6, **characterized in that** the protein is a protein hydrolysate obtained from soya or wheat.

8. Granules according to any one of Claims 1 to 7, **characterized in that** the dispersant (D) is anionic and chosen from water-soluble salts of alkyl sulphates or of alkyl ether sulphates, alkyl isethionates and alkyl taurates or salts thereof, alkyl carboxylates, alkyl sulphosuccinates or alkyl succinamates, alkyl sarcosinates, alkyl derivatives of protein hydrolysates, acyl aspartates, and alkyl and/or alkyl ether and/or alkylaryl ether ester phosphates, lignosulphonates, polynaphthalenesulphonates, copolymers comprising units derived from at least one monomer chosen from unsaturated C₃-C₅ acids, diacids or anhydrides and units derived from at least one unsaturated, linear or branched C₄-C₈ hydrocarbon.

9. Granules according to any one of Claims 1 to 7, **characterized in that** the dispersant (D) is cationic and chosen from alkyldimethylbenzylammonium halides and alkyldimethylethylammonium halides.

10. Granules according to any one of Claims 1 to 7, **characterized in that** the dispersant (D) is amphoteric and chosen from alkylbetaines, alkyldimethylbetaines, alkylamidopropylbetaines, alkylamidopropyldimethylbetaines, alkyltrimethylsulphobetaines, imidazoline derivatives such as alkyl amphoacetates, alkyl amphodiacetates, alkyl amphopropionates, alkyl amphodipropionates, alkylsultaines or alkylamidopropylhydroxysultaines, the condensation products of fatty acids and of protein hydrolysates, amphoteric derivatives of alkylpolyamines, and proteins or protein hydrolysates.

11. Granules according to Claim 10, **characterized in that** the dispersant (D) is a protein or a protein hydrolysate.

12. Granules according to any one of Claims 1 to 11, **characterized in that** up to 95%, preferably up to 50%, of the weight of the protein (PP) of plant origin constituting the matrix is replaced with a water-soluble or water-dispersible ose, oside or polyholoside (O) or a polyelectrolyte (PE) belonging to the family of weak polyacids.

13. Two-step process for preparing water-dispersible granules forming the subject of Claim 1,
- the first step consisting in preparing a dispersion in water comprising at least one hydrophobic active material, at least one ionic or amphoteric dispersant (D) and at least one water-soluble or water-dispersible protein (PP) of plant origin,
- the second step consisting in drying the said dispersion until a granulate forms.

14. Process according to Claim 13, **characterized in that** the relative amounts of hydrophobic active material (AM), of ionic or amphoteric dispersant (D) and of water-soluble or water-dispersible protein (PP) of plant origin, used are such that the said dispersion, expressed as dry material, comprises
- from 5% to 90%, preferably from 40% to 85% and most particularly from 50% to 80%, of its weight of organic hydrophobic active material (AM),
- from 3% to 90%, preferably from 10% to 60% and most particularly from 15% to 50%, of its weight of water-soluble or water-dispersible protein (PP) of plant origin
- from 0.02% to 20%, preferably from 0.1% to 10%, of its weight of ionic or amphoteric dispersant (D), the said percentages being expressed by weight of solids.

15. Process according to Claim 13 or 14, **characterized in that** the amount of solids in the dispersion is between 10% and 70% by weight and preferably between 30% and 60% by weight.

16. Process according to any one of Claims 13 to 15, **characterized in that** the dispersion is a suspension of solid active material (AM) whose mean particle size is between 1 µm and 50 µm, preferably between 1 µm and 5 µm, and which has a viscosity (Brookfield, 20 rpm at 25°C) of less than 1000 mPa.s.

17. Process according to any one of Claims 13 to 15, **characterized in that** the dispersion is an emulsion of liquid active material (AM), whose mean particle size is between 0.11 µm and 10 µm, preferably between 0.2 µm and 5 µm.

18. Process according to any one of Claims 13 to 17, **characterized in that** the hydrophobic organic active material (AM), the water-soluble or water-dispersible protein (PP) of plant origin, and the ionic or amphoteric dispersant (D) are chosen from those mentioned in Claims 5, 6, 7, 8, 9, 10, 11 and 12.

19. Process according to any one of Claims 13 to 18, **characterized in that** the drying of the dispersion is carried out by lyophilization or by spray-drying.

20. Process according to any one of Claims 13 to 18, **characterized in that** anticaking agents are introduced during the drying step.

## Patentansprüche

1. Wasserdispergierbare Granulate, umfassend:
- mindestens einen hydrophoben organischen Aktivstoff (MA), der fest ist oder einen Schmelzpunkt unterhalb von 100 °C besitzt, fein verteilt in oder verkapselt mittels einer feste Matrix in einem wasserlöslichen oder wasserdispergierbaren Protein (PP) pflanzlichen Ursprungs; und
- mindestens ein ionisches oder amphoteres Dispergiermittel (Dispergator) (D) an der Grenzfläche Aktivstoff/Matrix.

2. Granulate nach Anspruch 1, **dadurch gekennzeichnet, daß** sie enthalten:
- 5 bis 90 %, vorzugsweise 40 bis 85 %, insbesondere 50 bis 80 %, ihres Gewichts an organischem hydrophobischem Aktivstoff (MA),
- 3 bis 90 %, vorzugsweise 10 bis 60 %, insbesondere 15 bis 50 %, ihres Gewichts an wasserlöslichem oder wasserdispergierbarem Protein (PP),
- 0,02 bis 20 %, vorzugsweise 0,1 bis 10 %, ihres Gewichts an ionischem oder amphoterem Dispergiermittel (D),
wobei die Prozentangaben als Gewicht des Trockenmaterials berechnet sind.

3. Granulate nach Anspruch 1 oder 2, **dadurch gekennzeichnet, daß** die Teilchen des Aktivstoffes (MA) eine mittlere Granulometrie (Teilchengröße) in der Größenordnung von 0,1 bis 50 µm, vorzugsweise in der Größenordnung von 0,1 bis 10 µm, insbesondere 0,2 bis 5 µm, aufweisen.

4. Granulate nach einem der Ansprüche 1 bis 3, **dadurch gekennzeichnet, daß** der hydrophobe organische Aktivstoff bei herkömmlicher Temperatur flüssig ist.

5. Granulate nach einem der Ansprüche 1 bis 4, **dadurch gekennzeichnet, daß** der hydrophobe organische Aktivstoff ein Aktivstoff aus dem Lebensmittelbereich, dem Kosmetikbereich, dem Bereich der Lacke und Farben, dem Papierbereich, dem Bereich der Wasch- bzw. Reinigungsmittel, dem Bereich der Baumaterialien oder dem Bereich der phytosanitären Aktivstoffe (Pflanzenschutzmittel) ist oder ein Schmiermittel für die Bearbeitung oder Umformung bzw. Formgebung von Materialien ist.

6. Granulate nach einem der Ansprüche 1 bis 5, **dadurch gekennzeichnet, daß** das Protein (PP) ein hydrolisiertes Protein pflanzlichen Ursprungs mit einem Hydrolysegrad von weniger als 40 % ist.

7. Granulate nach Anspruch 6, **dadurch gekennzeichnet, daß** das Protein ein Proteinhydrolysat aus Soja oder Weizen bzw. Getreide ist.

8. Granulate nach einem der Ansprüche 1 bis 7, **dadurch gekennzeichnet, daß** das Dispergiermittel (D) anionisch ist und ausgewählt ist aus wasserlöslichen Salzen von Alkylsulfaten, Alkylethersulfaten, Alkylisothionaten und Alkyltauraten oder ihren Salzen, Alkylcarboxylaten, Alkylsulfosuccinaten oder Alkylsuccinamaten, Alkylsarkosinaten, alkylierten Derivaten von Proteinhydrolysaten, Acylaspartaten, Alkyl- und/oder Alkylether- und/oder Alkylaryletherphosphatestern, Lignosulfonaten, Polynaphthalinsulfonaten und Copolymeren mit Einheiten, die sich ableiten von mindestens einem Monomer, ausgewählt aus C₃-C₅-Säuren, -disäuren oder ungesättigten -Anhydriden, und Einheiten, die sich ableiten von mindestens einem linearen oder verzweigten ungesättigten C₄-C₈-Kohlenwasserstoff.

9. Granulate nach einem der Ansprüche 1 bis 7, **dadurch gekennzeichnet, daß** das Dispergiermittel (D) kationisch ist und ausgewählt ist aus Alkyldimethylbenzylammoniumhalogeniden und Alkyldimethylethylammoniumhalogeniden.

10. Granulate nach einem der Ansprüche 1 bis 7, **dadurch gekennzeichnet, daß** das Dispergiermittel (D) amphoter ist und ausgewählt ist aus Alkylbetainen, Alkyldimethylbetainen, Alkylamidopropylbetainen, Alkylamidopropyldimethylbetainen, Alkyltrimethylsulfobetainen, Imidazolinderivaten wie Alkylamphoacetaten, Alkylamphodiacetaten, Alkylamphopropionaten, Alkylamphodipropionaten, Alkylsultainen und Alkylamidopropylhydroxysultainen, Kondensationsprodukten von Fettsäuren und Proteinhydrolysaten, amphoteren Derivaten von Alkylpolyaminen, Proteinen und Proteinhydrolysaten.

11. Granulate nach Anspruch 10, **dadurch gekennzeichnet, daß** das Dispergiermittel (D) ein Protein oder ein Proteinhydrolysat ist.

12. Granulate nach einem der Ansprüche 1 bis 11, **dadurch gekennzeichnet, daß** bis zu 95 %, vorzugsweise bis zu 50 %, des Gewichts des Proteins pflanzlichen Ursprungs (PP), welches die Matrix bildet, ersetzt ist durch eine Ose, ein Osid oder ein wasserlösliches oder ein wasserdispergierbares Polyholosid (O) oder einen Polyelektrolyt (PE) aus der Familie der schwachen Polysäuren.

13. Verfahren zur Herstellung der wasserdispergierbaren Granulate gemäß Anspruch 1 in zwei Verfahrensstufen, wobei
- die erste Verfahrensstufe darin besteht, eine Dispersion in Wasser herzustellen, die mindestens einen hydrophoben Aktivstoff, mindestens ein ionisches oder amphoteres Dispergiermittel (D) und mindestens ein wasserlösliches oder wasserdispergierbares Protein (PP) pflanzlichen Ursprungs enthält, und
- die zweite Verfahrensstufe in der Trocknung dieser Dispersion bis zum Erhalt eines Granulats besteht.

14. Verfahren nach Anspruch 13, **dadurch gekennzeichnet, daß** die jeweiligen eingesetzten Mengen an hydrophobem Aktivstoff (MA), ionischem oder amphoterem Dispergiermittel (D) und wasserlöslichem oder wasserdispergierbarem Protein pflanzlichen Ursprungs (PP) derart sind, daß die Dispersion, berechnet als Trockengewicht, umfaßt:
- 5 bis 90 %, vorzugsweise 40 bis 85 %, insbesondere 50 bis 80 %, ihres Gewichts an organischem hydrophobem Aktivstoff (MA),
- 3 bis 90 %, vorzugsweise 10 bis 60 %, insbesondere 15 bis 50 %, ihres Gewichts an wasserlöslichem oder wasserdispergierbarem Protein pflanzlichen Ursprungs (PP),
- 0,02 bis 20 %, vorzugsweise 0,1 bis 10 %, ihres Gewichts an ionischem oder amphoterem Dispergiermittel (D),
wobei die Prozentangaben als Gewicht des Trockenmaterials berechnet sind.

15. Verfahren nach Anspruch 13 oder 14, **dadurch gekennzeichnet, daß** die Menge an Trockenmaterial der Dispersion 10 bis 70 Gew.-%, vorzugsweise 30 bis 60 Gew.-%, beträgt.

16. Verfahren nach einem der Ansprüche 13 bis 15, **dadurch gekennzeichnet, daß** die Dispersion eine Suspension des festen Aktivstoffs (MA) ist, wobei die mittlere Teilchengröße 1 µm bis 50 µm, vorzugsweise 1 µm bis 5 µm, beträgt und die Viskosität (Brookfield, 20 Upm bei 25°C) kleiner als 1.000 mPa·s ist.

17. Verfahren nach einem der Ansprüche 13 bis 15, **dadurch gekennzeichnet, daß** die Dispersion eine Emulsion des flüssigen Aktivstoffs (MA) ist, wobei die mittlere Teilchengröße 0,11 µm bis 10 µm, vorzugsweise 0,2 µm bis 5 µm, beträgt.

18. Verfahren nach einem der Ansprüche 13 bis 17, **dadurch gekennzeichnet, daß** der hydrophobe organische Aktivstoff (MA), das wasserlösliche oder wasserdispergierbare Protein pflanzlichen Ursprungs (PP) und das ionische oder amphotere Dispergiermittel (D) aus den Verbindungen ausgewählt sind, die in den Ansprüchen 5, 6, 7, 8, 9, 10, 11 oder 12 genannt sind.

19. Verfahren nach einem der Ansprüche 13 bis 18, **dadurch gekennzeichnet, daß** die Trocknung der Dispersion durch Lyophilisierung (Gefriertrocknung) oder durch Atomisieren (Zerstäuben) erfolgt.

20. Verfahren nach einem der Ansprüche 13 bis 18, **dadurch gekennzeichnet, daß** während des Trocknungsschrittes verklumpungsverhindernde Mittel zugegeben werden.
